# EUROPEAN PATENT SPECIFICATION

(11) **EP 4 003 453 B1**
(45) Date of publication and mention of the grant of the patent: **07.01.2026**
(21) Application number: 20843366.4
(22) Date of filing: 23.07.2020
(51) Int. Cl.: A61M 5/34, A61M 5/20, G01P 5/26, G01F 1/704, A61M 5/315, G01F 1/684, G01F 1/692, G01F 15/18, G01P 5/18, G01F 15/075, A61M 5/168, G01F 22/00

(54) **IMPROVED FLOW CHANNEL FOR FLOW RATE MEASUREMENT**
VERBESSERTER DURCHFLUSSKANAL FÜR DURCHFLUSSRATENMESSUNG
CANAL D'ÉCOULEMENT AMÉLIORÉ POUR MESURE DE DÉBIT

(30) Priority: 25.07.2019 US 201962878382 P
(43) Date of publication of application: 01.06.2022
(73) Proprietor: Becton, Dickinson and Company, Franklin Lakes, NJ 07417-1880 (US)
(72) Inventor: PETISCE, James R., Westford, Massachusetts 01886 (US); MAUL, Christopher, Franklin Lakes, New Jersey 07417 (US); METTERS, Andrew T., Franklin Lakes, New Jersey 07417 (US)
(74) Representative: dompatent
(86) International application number: PCT/US2020/043254
(87) International publication number: WO 2021/016446

(56) References cited:
- CN-U- 203 090 111
- US-A1- 2018 036 495
- US-A1- 2019 167 919
- US-A1- 2019 167 919
- US-B1- 6 668 643

## Description

### Field of the Invention

The present invention relates to medication delivery devices. In particular, the present invention relates to improved systems for measuring a dose delivered from an injection pen, or the like, utilizing a flow sensor within an improved flow channel.

### Background of the Invention

Insulin injection is a required daily task in the lives of people afflicted with diabetes. The action of insulin injection deceptively appears to be a simple task, but it is fraught with a number of risks to the insulin using patient. A patient is routinely requested to maintain a written diary containing the time of the insulin injection and the amount of insulin injected. Maintenance of such a written diary is made difficult by several confounding factors. When an insulin injection is made, the patient may be pre-occupied with another activity such as attending a meeting or participating in a telephone conversation which makes writing an entry into the diary difficult or impossible. Additionally, a consequence of chronic hyperglycemia commonly experienced by people with diabetes is cognitive impairment which renders a patient unable to remember when an insulin dose was taken and/or what dose was actually injected. Accordingly, what is needed is an attachment to an insulin pen which automatically measures the amount of insulin injected and what time the insulin was injected. Such a device solves the inability to make entries into the written diary described above and renders such a written diary as unnecessary or obsolete.

Accurate dose measurement is an important component of any medicine therapy, and especially important for an insulin therapy regimen for diabetics. Recently, a few injection pens and pen attachments have been developed for the purpose of measuring and automatically data logging the dose delivered, e.g. motorized injection pens and attachments that approximate the position of a plunger within an insulin reservoir to determine how much insulin has been delivered. However, none of the current solutions are adequate. Manual recording of insulin doses is inherently inaccurate due to human errors and omissions, and measurement of a plunger, while an improvement over manual recording, is still not accurate enough for individual doses and does not record the time that doses are delivered.

Thermal time of flight (TTOF) sensors have been used to detect the time of flight of a heat pulse induced into moving fluid as it travels through a channel of known cross-section over a known distance in order to measure volumetric flow of the fluid. However, existing TTOF sensors are typically used in steady state flow scenarios, and have not to date been required to measure rapid and large changes in flow rate, as is expected in an insulin injection from an insulin pen, or the like.

An insulin pen attachment has been developed which is an injection molded plastic part into which is placed a chip to measure the flow of insulin out of the insulin pen then into the insulin pen needle and ultimately into the subcutaneous tissue of the insulin using patient. An exemplary insulin pen attachment utilizing a TTOF sensor is described in greater detail in U.S. Patent No. 10,052,441. The accuracy of such sensors are dependent on the accuracy of the cross sectional area of the flow channel in which the sensor is housed. Although an injection molded part has its dimensions such as internal diameter and roundness specified, the injection molding process is limited in its ability to achieve precise consistency from one molded part to another. Common production tolerances that can be achieved economically depend on the types of polymer and fillers used can easily exceed +/- 0.005". Finer tolerances approaching+/- 0.001" can be achieved with some polymers, but at a significant incremental cost. Such variability translates into variability in dimensions of the insulin pen attachment to such an extent that each individual attachment would need to be calibrated before insulin pen attachment to ensure accurate measuring performance of the attachment. Individual calibration of each insulin pen attachment is cumbersome and expensive for the manufacturer of such an attachment. Accordingly, there is a need for an improved flow channel to house a flow sensor within an insulin pen attachment, to improve accuracy, lower the need for and costs associated with calibration, and the like.

US 2019/0167919 A1 discloses a flow sensor provided to enable volumetric dose data to be acquired automatically by sampling flow rates of insulin measured by a flow sensor exposed to a flow manifold through which the insulin flows.

CN 203090111 U discloses a measuring type injector.

### Summary of the Invention

The above described disadvantages are overcome and other advantages are realized by embodiments of the present invention, as will be appreciated by those of ordinary skill in the art. The present invention is defined by the appended patent claims.

Exemplary embodiments of the invention provide an improved liquid channel assembly to measure insulin flow as it moves out of an insulin pen and into an insulin pen needle and then ultimately into a patient. Embodiments of the invention replace the plastic assembly formed by injection molding with a metallic cannula or elongated needle. The metallic cannula or needle is provided with a window where a flow measurement chip is positioned. Metallic cannula or elongated needles are advantageously able to be mass produced from welded or seamless tubes from metal sheet. Such processes are advantageously capable of holding tolerances on the inside diameter of less than +/- 0.00075". Consequently,a metallic cannula-based flow channel can be produced at low cost with the sufficient part to part consistency required to eliminate the need for individual calibration of each flow channel assembly. This simplifies the related manufacturing processes.

Additionally, the inner surface of such a metallic-based insulin flow path provides superior consistency of the surfaces presented to insulin flowing through it as compared to the surface of an injected molded part. Such an improved surface finish consistency improves part to part consistency of fluid flow through the flow channel. Fluid flowing through such a channel can either be laminar or turbulent in nature which can affect the quality of the flow sensing measurement capabilities of the flow sensing chip.

Another important attribute of the flow channel is insulin compatibility which can be affected by the surface roughness of the flow channel. Specifically, the fractal length of the flow channel surface can provide sites for nucleation or deposition of the insulin as it transitions through the flow path. A metallic-based flow channel could be electropolished if necessary to present a contact surface with minimal fractal length as compared to an injection molded surface.

### Brief Description of the Drawings

The invention will be more readily understood with reference to the embodiments thereof illustrated in the attached drawing figures, in which:
FIG. 1 illustrates a dose capture system according to an exemplary embodiment of the present invention;
FIG. 2 illustrates a semi-disposable portion of the dose measuring system of FIG. 1;
FIG. 3 illustrates a durable portion of the dose measuring system of FIG. 1;
FIG. 4 is a block diagram of a dose capture system according to an exemplary embodiment of the invention;
FIG. 5 is a cross sectional view of a semi-disposable portion of a dose measurement system utilizing an improved flow channel according to an exemplary embodiment of the invention;
FIG. 6 illustrates elevation and perspective views of an improved flow channel according to an exemplary embodiment of the invention;
FIG. 7 is a flow chart illustrating a method of forming a semi-disposable portion of a dose capture system according to an exemplary embodiment of the invention;
FIGS. 8A-8C illustrate exemplary user interfaces for a dose capture system according to an exemplary embodiment of the invention;
FIG. 9 illustrates a dose profile captured by a dose capture system according to an exemplary embodiment of the invention;
FIG. 10 illustrates a cross section of a static thermal field in a sensor with fluid not flowing;
FIG. 11 illustrates a cross section of a distorted thermal field in a sensor with fluid flowing;
FIG. 12 illustrates a sum of trapezoids method of integrating flow readings to calculate a dose volume according to an exemplary embodiment of the invention; and
FIG. 13 illustrates an alternate embodiment of the invention utilizing two flow sensors to improve dynamic range of a dose capture system according to an exemplary embodiment of the invention.

Throughout the drawings, like reference number should be understood to refer to like elements, features and structures.

### Detailed Description of the Exemplary Embodiments

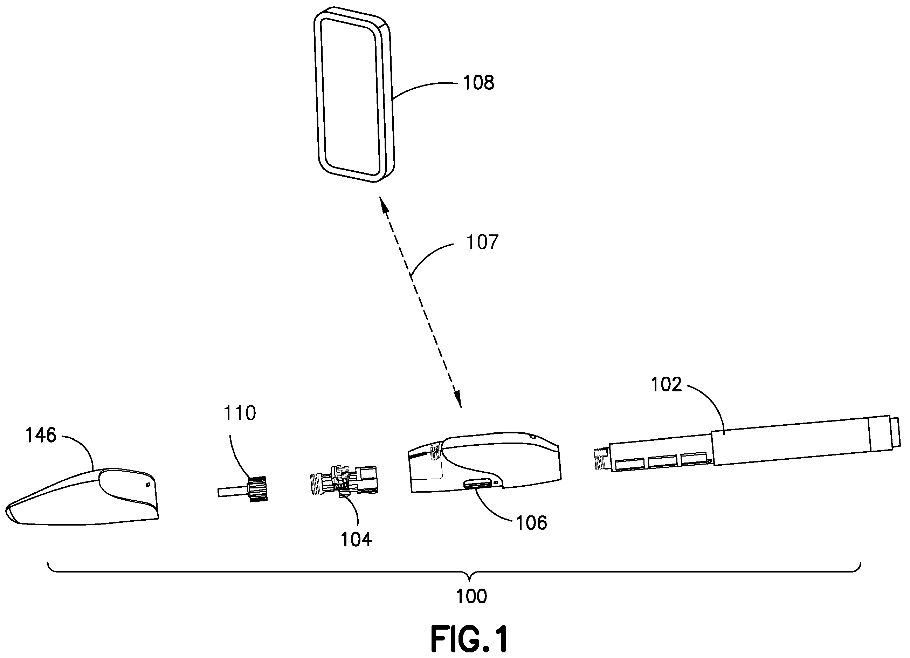

The exemplary embodiments of the invention will now be described with reference to the attached drawing figures. FIG. 1 illustrates an exemplary dose capture system 100 that preferably integrates with a conventional insulin pen 102. While this exemplary embodiment is illustrated in connection with an insulin pen, it should be appreciated that embodiments of the invention may be utilized with any suitable medication device, including without limitation, patch pumps, IV pumps, fixed dose injectors, auto injectors, syringes, and so on. The system 100 comprises a semi-disposable flow sensor 104 that includes a fluid manifold and a Thermal Time of Flight (TTOF) hybrid sensor. The semi-disposable flow sensor 104 preferably has a life equivalent to the insulin pen 102 to which it is attached. The system 100 further comprises a durable portion 106, which preferably has a multi-year lifespan. The durable portion 106 consists of a plastic enclosure containing electronics that power the flow sensor and read the sensor signals, a micro-processor for analyzing dose data, re-chargeable battery, temperature and motion and/or position sensors, and wireless communication circuitry. The durable portion 106 also preferably has removable cap 146 that can provide one or more of the following functions: protects the semi-disposable flow sensor, shields the insulin from light, protects the patient from unintended needle stick, and acts as a switch for an electrical contact closure that activates and deactivates the sensing system, when the cap 146 is removed from or replaced onto the durable, respectively. The durable portion 106 preferably is adapted to be charged via a standard connector such as a USB port, or preferably, via a wireless charging system. Preferably, a smart phone 108 based software application interacts wirelessly with the durable portion 106 to read, store, and present dose information. The wireless communication between the durable portion 106 and the smart phone 108 is indicated by the dashed line 107 in Fig. 1. The application can also interact with other electronic devices and networks, such as glucose meters, activity & fitness meters, or diabetes care networks. The software application is preferably paired with the durable portion 106 one time. After the initial pairing, the software automatically recognizes the durable portion 106 and is capable of securely transferring data from the durable portion 106 to the smart phone application automatically. It should be appreciated that in alternate embodiments of the invention, other pairing arrangements could be made as appropriate and desired.

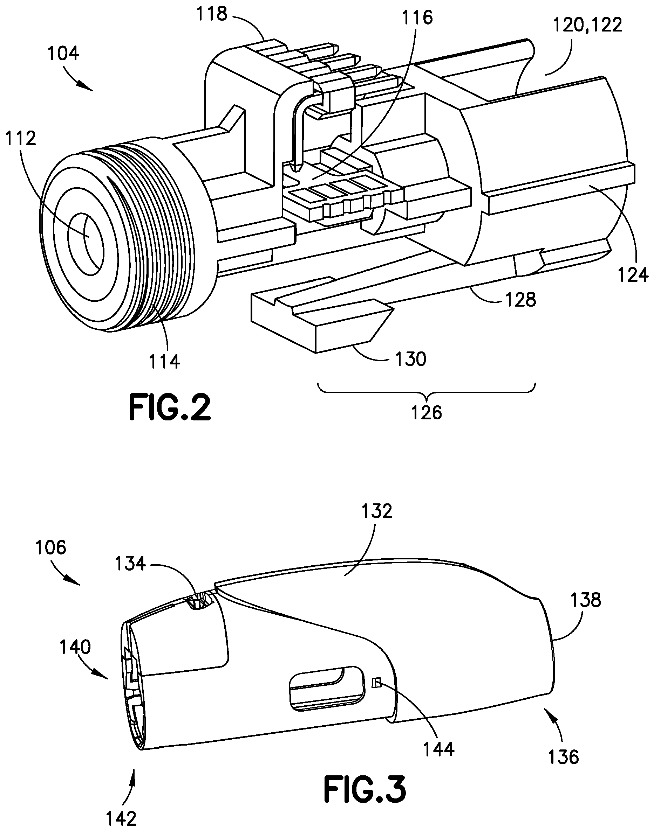

As illustrated in FIG. 2, the semi-disposable flow sensor 104 preferably has a threaded portion 114 to receive a standard insulin pen needle 110. The pen needle 110 is preferably changed with each insulin dose as is conventional.

The semi-disposable flow sensor 104 will now be described in further detail in connection with FIG. 2. As illustrated, a distal end of the semi-disposable flow sensor 104 comprises a septum 112 and a universal pen needle thread 114. A MEMS flow sensor chip 116 is mounted on a carrier printed circuit board, and fixed to the semi-disposable flow sensor assembly 104. An electrical connector 118 is provided for making electrical connections between the semi-disposable flow sensor portion 104 and the durable portion 106. A proximal end of the semi-disposable flow sensor portion 104 comprises an insulin pen connection 120 with an inlet cannula 122. The semi-disposable 104 preferably also includes alignment features 124 on the housing to align the semi-disposable 104 within the durable portion 106. The semi-disposable preferably includes an axial lock 126, or similar feature, to releasably lock the semi-disposable 104 within the durable portion 106. As shown, the axial lock 126 comprises a flexible member 128 and a locking member 130 adapted to be locked into a corresponding feature of the durable portion 106.

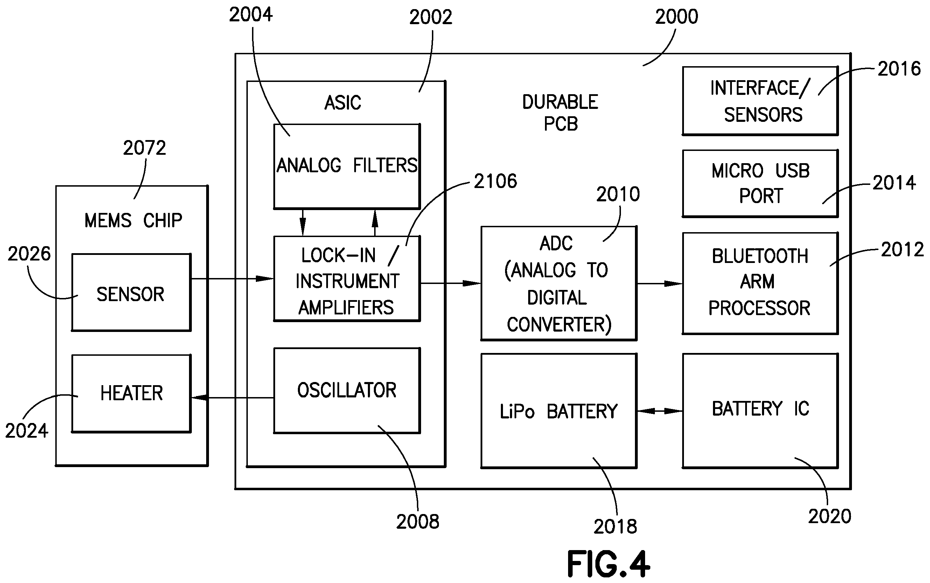

The durable portion 106 will now be described in further detail with reference to FIGS. 3 and 4. As illustrated, the durable portion 106 comprises an outer housing 132 that preferably includes an opening for a charging port 134. The charging port 134 preferably conforms to a regularly adopted standard such as mini-USB, or the like, although any suitable connection may be utilized, including a proprietary connection. Alternately, a wireless charging arrangement can be incorporated into the durable unit. The durable unit has a proximal end 136 that includes an opening 138 for attachment to an insulin pen 102. The durable unit further has a distal end 140 that includes an opening 142 adapted to receive the semi-disposable portion 104. The durable potion 106 preferably is contoured and includes detents 144, or similar features, to receive a removable cap 146. The durable portion 106 includes a printed circuit board 2000, illustrated in FIG. 4. The durable PCB 2000 preferably includes an ASIC 2002, that provides analog filters 2004, lock-in/instrument amplifiers 2006, and an oscillator 2008. The ASIC 2002 provides data to an analog to digital converter (ADC) 2010. The ADC 2010 in turn provides data to a Bluetooth ARM processor 2012. The durable PCB 2000 further includes a communications port 2014 such as a micro-USB port, interface/sensors 2016, and battery components 2018/2020. The durable PCB 2000 interfaces with the MEMS chip 2022 that provides the heater 2024, and sensor elements 2026.

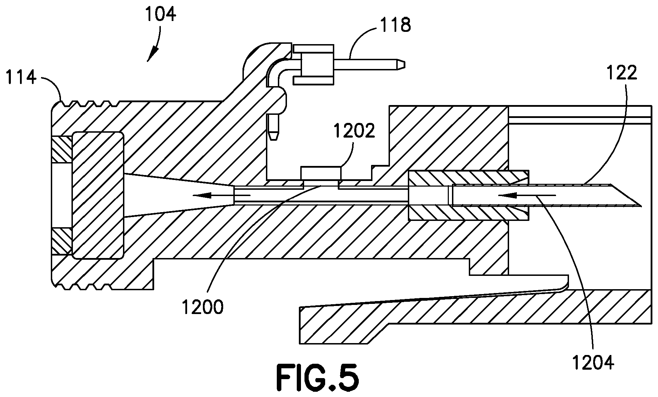

FIG. 5 is a cross sectional view of the semi-disposable flow sensor 104. FIG. 5 shows the sensor chip 1200 mounted onto a PCB 1202 with an exposed sensor surface within a flow manifold 1204. Metal cannula 1206 is formed inside the flow manifold 1204. Because metal cannulas may be formed with more precise geometry, including inside diameter, the overall accuracy of the sensor is improved. Metallic cannula or elongated needles are advantageously able to be mass produced from welded or seamless tubes from metal sheet. These tubes are then reduced by use of several intervening annealing and drawing steps until final dimensions are achieved. Advantageously, calibration of sensors so formed may be simplified or eliminated because of the reduction in sensor to sensor variability. The chip 1200 may be mounted and sealed to the manifold 1204 by any suitable means including UV cured adhesive, molded elastomeric seals in a gland or an over-molded elastomeric seal. One end of the manifold 1204 contains a piercing cannula 122 and attaches to the ISO standard hub of an insulin pen. It should be appreciated that the piercing cannula 122 need not be a separate part from metal cannula 1206, and may be integrally formed therewith. Attaching the manifold 1204 to the pen 102 punctures the rubber septum on the insulin cartridge and establishes an insulin flow path over the sensor 1200. The opposite end of the manifold 1204 contains threads and an elastomeric septum that create an ISO compliant connection for insulin pen needles. The manifold 1204 is formed to have minimum residual (unrecoverable) internal volume, ideally less than 30 microliters, and minimum added length, ideally less than 25mm. The manifold 1204 includes a metal flow channel 1206 designed with a cross sectional area and smooth transitions to ensure that laminar flow is maintained at all times over the sensor 1200 face. The sensor 1200 face is positively positioned with respect to the channel wall so that it is always in the shear zone of the insulin flow. Sensor face positioning should be 0mm to 0.1mm proud with respect to the manifold wall, with 0.05 mm preferred. The metal flow channel 1206 through the manifold 1204 is preferably formed in a substantially straight line from inlet cannula 122 to sensor surface 1200 and on to the pen needle end to promote laminar flow of insulin through the flow channel 1206.

The manifold 1204 preferably has alignment features that ensure proper orientation and positioning of the semi-disposable portion 104 relative to the durable portion 106 during insertion and set up. A retention feature such as a snap flexure 1208 secures the semi-disposable portion 104 to the durable portion 106 and the insulin pen 102 during use and allows the user to release and remove the semi-disposable when the pen 102 is empty. An electrical connector 118 on the manifold 1204 is preferably oriented in the same direction as the inlet cannula 122 and establishes electrical contact with the durable portion 106 at the same time the flow path is being established when inserting the semi disposable portion 104 into the durable portion 106. The electrical connector 118 shown in FIG. 5 has conductive pins, but other features such as conductive pads, flexible cables, conductive flexures, or pins could also be used.

For a given sensing chip with an established heater to sensor spacing, the measurable flow range can be adjusted by changing the flow channel cross section. For a given flow rate, a larger cross section will reduce the apparent velocity observed by the chip, allowing the chip to measure higher flow rates before the sensor signal saturates. The larger cross section will have an inherent trade off of reduced accuracy for low flow rates. A smaller cross section can be paired with a larger element spacing to measure an equivalent flow range with reduced internal volume in the flow path.

The semi-disposable 104, and more specifically the elements comprising the flow channel 1206 are preferably designed with materials that are compatible and non-binding with insulin for the full life of the pen injector, that is, up to at least 28 days. Such materials include ABS plastic and 304 series stainless steel, among others. If liquid silicone rubber is used for the seal between the PCB and the manifold, and since elastomers have tendency to adsorb the preservative from the insulin, the exposed surface of the rubber seal is minimized. Medical grade light cured adhesives are used to bond manifold components. These preferably include flash cured cyanoacrylates or light cured acrylics.

The insulin flow channel 1204 is designed with gradual flow transitions in order to avoid any zones of high shear, which could potentially damage the insulin protein molecules. The manifold threaded hub 114 is preferably designed to accept ISO standard insulin pen needles.

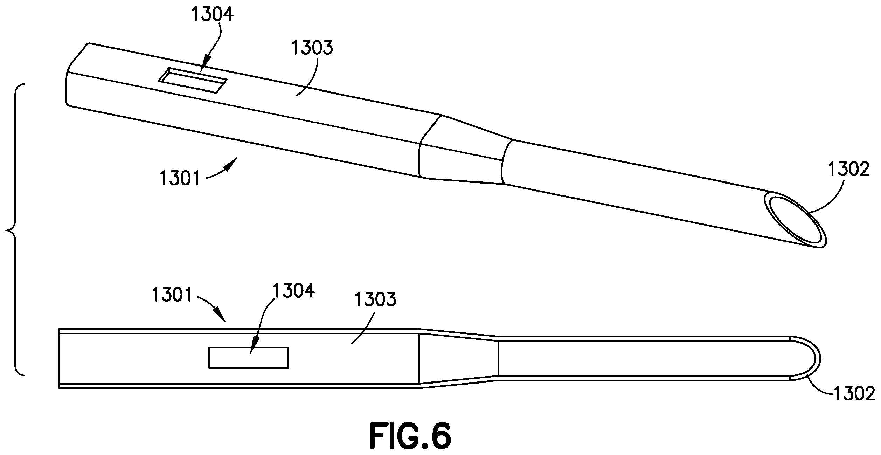

The semi-disposable 104 illustrated in FIG. 4 is preferably an injection molded thermoplastic component formed around metal cannula 1206. FIG. 6 illustrates a metal cannula 1301 according to an exemplary embodiment that includes both the piercing portion 1302 and the flow channel portion 1303. As illustrated a sensor window 1304 is formed in the flow channel portion 1303 . The flow rate sensor is placed in the sensor window 1304 to access fluid flowing within the flow channel. Portions of the flow channel 1204 of the semi-disposable 104 could be replaced with metal, plastic, ceramic, or composite tubing 1301 that has been micro-fabricated to provide the smooth flow transitions described above. However, for accuracy, it is preferred that the portion of the flow channel in which the sensor window 1304 is located is formed of a metal cannula. The metallic flow channel 1301 is preferably insert-molded and incorporated into the semi-disposable 104, or the plastic portion of semi-disposable 104 is injection molded around the metal flow channel.

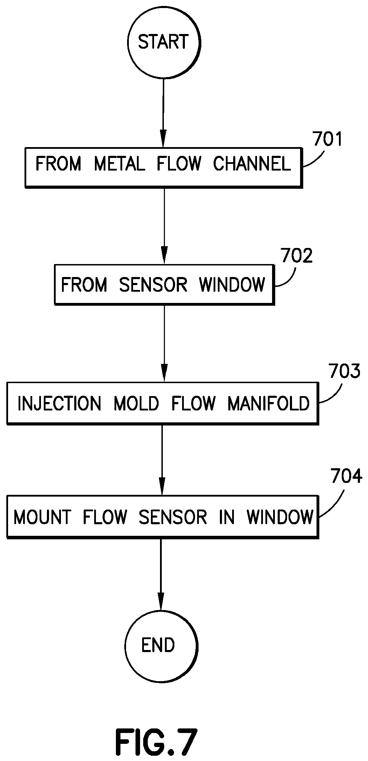

A method of fabricating a flow sensor for use with an insulin pen will now be described in connection with FIG. 7. At step 701, a metal flow channel is formed with a predetermined cross sectional area. Needle manufacturing techniques are preferably used to take advantage of high precision diameter. A proximal end of the metal flow channel optionally includes a piercing member. At step 702 a sensor window is formed between the ends of the metal flow channel. At step 703 a flow manifold body is injection molded around the metal flow channel. The flow manifold body includes a proximal end adapted to expose the piercing member, and shaped to connect to an insulin pen. The flow manifold distal end includes a threaded outer portion adapted to receive a pen needle, and is shaped to receive a septum therein. At step 704, a flow sensor is mounted against the window in the flow channel.

Now operation of the dose capture system according to an exemplary embodiment will be described. The dose capture system 100 is installed on an insulin pen 102 as part of the set up sequence with each new pen, that is, every three (3) to seven (7) days (nominally five (5)) for a typical user. The durable portion 106 is first attached to the insulin pen 102. The semi-disposable 104 is then inserted into the distal opening 140 of the durable portion 106. Cannula 122 of the disposable portion 104 pierces the distal septum of the insulin pen 102, creating a flow path over the TTOF sensing element. As the semi-disposable portion 104 is inserted into the durable portion 106, electrical connector 118 is mated with a corresponding electrical connector within the durable portion 106, creating electrical connections to the TTOF sensor 116. A pen needle is threaded onto the distal threaded end 114 if the disposable portion 104, such that the pen needle cannula pierces the septum 112, completing a fluid path from the insulin pen through the flow sensor and pen needle. The combined insulin pen and dose sensing system is then primed in the normal manner to remove trapped air.

Although in this embodiment the assembly sequence is durable portion 106 first and semi-disposable portion 104 second, the system can be designed with the assembly order reversed, as will be appreciated by those of ordinary skill in the art. With this assembly sequence, the durable portion 106 could be used on different insulin pens, such as one pen with slow acting insulin and a second pen with fast acting insulin. The semi-disposable portion 104 preferably attaches to the universal ISO connection found on each insulin pen, and the durable portion 106 would then attach to the semi-disposable portion 104 and to the body of the insulin pen. Since the durable portion 106 does not contact insulin, the durable portion 106 is capable of being swapped back and forth between multiple pens as required for the user's therapy without affecting the sterility of the insulin. For therapy involving more than one insulin or drug, a means of recognizing the additional drug to which the durable attached is provided. For example. a camera on the smart phone that is paired with the durable portion is used to read a bar code on the injection pen when the durable unit is attached to the pen.

The durable portion 106 is preferably paired with the smart phone application, as discussed above. A pairing procedure is preferably done once for a given cell phone 108/durable portion 106 pair. After the initial pairing, the cell phone 108 application preferably automatically recognizes and communication with the paired durable portion 106.

Once installed on the insulin pen, the exemplary system automatically recognizes and captures dose events as part of the user's normal injection sequence. Preferably, no additional use steps beyond those necessary for a normal insulin pen injection are required for the dose sensor after the initial set up on the pen. Dose volumes and times are calculated by the durable portion 106. The durable portion 106 preferably can store many insulin pens worth of dose data. Data recorded by the durable portion 106 is preferably transferred to the smart phone 108 application whenever the smart phone 108 and the durable portion 106 are within broadcast range of one another. The dose data transferred to the smart phone 108 is preferably presented to the user in a convenient and easy to read format. Dose information may also be transferred from the cell phone to other diabetes management devices or to a cloud based data storage site if desired for further processing and analysis and transfer to other stakeholders in the patient's healthcare network.

When the insulin pen 102 is empty, the durable portion 106 is removed and readied for the next use. The spent insulin pen 102 and the semi-disposable portion 104 combination are discarded in the same way as conventional diabetes pens. The durable portion 106 or the semi-disposable portion 104 preferably have features to prevent the reuse of a semi-disposable portion 104 on another insulin pen 102.

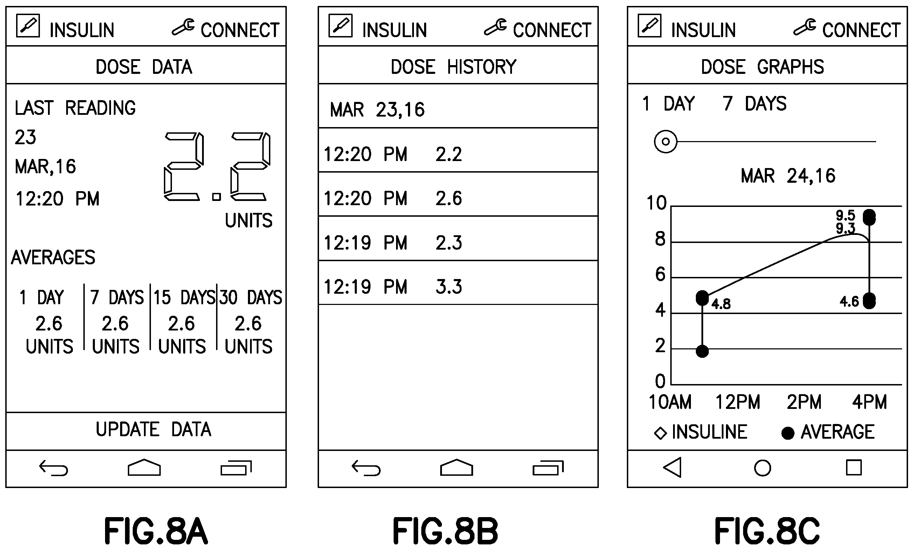

Next an exemplary smart phone 108 application will be described. The smart phone application preferably displays dose data to the user in an easy to understand format. Fig 8A shows a dose overview window, where recent dose measurements and time based averages can be shown. Fig 8B has a dose log that can be reviewed by the user for accuracy and also provides a location for notes and other context based data. Fig. 8C shows a graph of dose measurements over selectable time ranges, which can provide trend insights to the user.

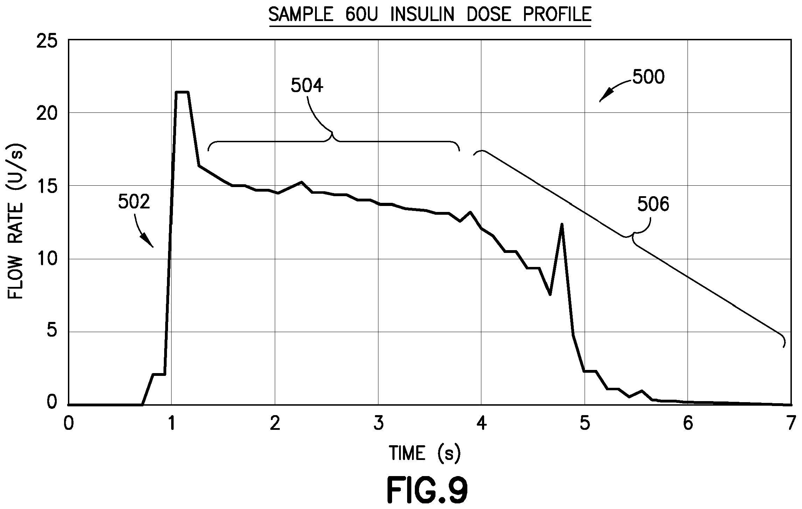

Several aspects of sulin injection present significant challenges to measuring accurate doses. Insulin dose size can vary greatly, from as low as 3.3 microliters to as high as 800 microliters. Dose delivery times can vary from less than 1 second to greater than 10 seconds based upon the size of the dose, diameter and length of the needle being used, the friction and mechanical efficiency of the insulin pen, and actuation force applied by the user or the spring loaded actuation of the pen. A typical insulin injection flow profile 500 is shown in FIG. 9. The flow profile has a sudden, large increase in flow at the beginning of the injection 502, a continuously varying flowrate 504, and relatively long flow decay 506 at the end of the dose. In order to calculate an accurate dose, the flow sensor must respond quickly and accurately to all portions of the flow profile. The flow sensor must be able to respond to the abrupt changes in flow and must be accurate over the range of flowrates that could be produced by a wide range of users. Advantageously, embodiments of the present invention are capable of determining a dose amount by integrating flow rate measurements over time. Alternately, several flow rate data points or an overall shape of the flow curve may be matched to a stored table of dose values, such as incremental doses from 1 unit to 60 units, for example

International standards currently require volumetric accuracy equivalent to the minimum resolution of the insulin pen or +/- 5% of the dose volume, whichever is greater. For example, on the typical U-100 pen with a dial resolution of 1U, accuracy of +/- 10 microliters for doses less than 200 microliters and +/- 5% for doses greater than 200 microliters is required. Higher insulin concentrations scale inversely with respect to volume. For instance, a U200 insulin pen with resolution of 1U would require volumetric accuracy of +/- 5 microliters for doses less than 100 microliters and +/- 2.5% for doses greater than 100 microliters.

Small diameter needles are typically used for insulin injections which can create relatively high back pressures. Accordingly, a flow sensor according to an embodiment of the invention must be able to tolerate back pressures of up to 1 mega-pascal.

Since the flow sensor is in the insulin delivery path, it must be manufactured from materials that are chemically compatible with the insulin, and must not in any way react with or break down the insulin.

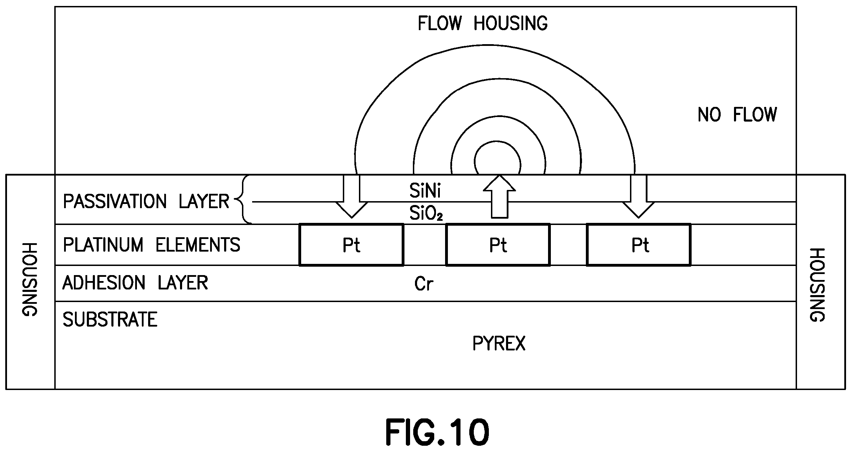

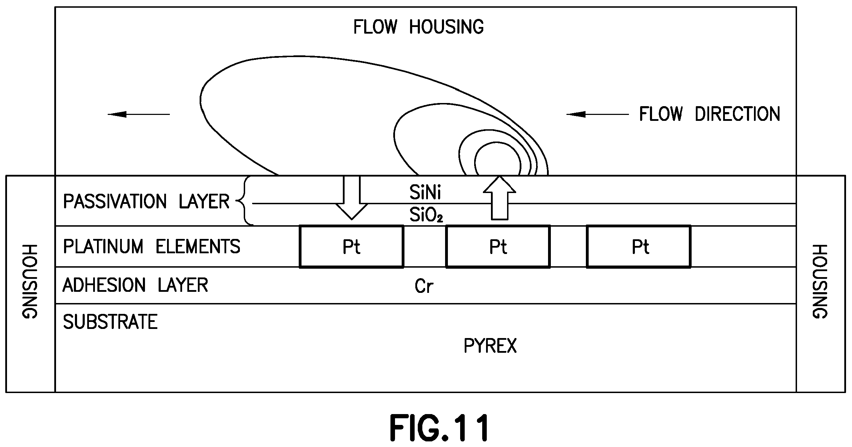

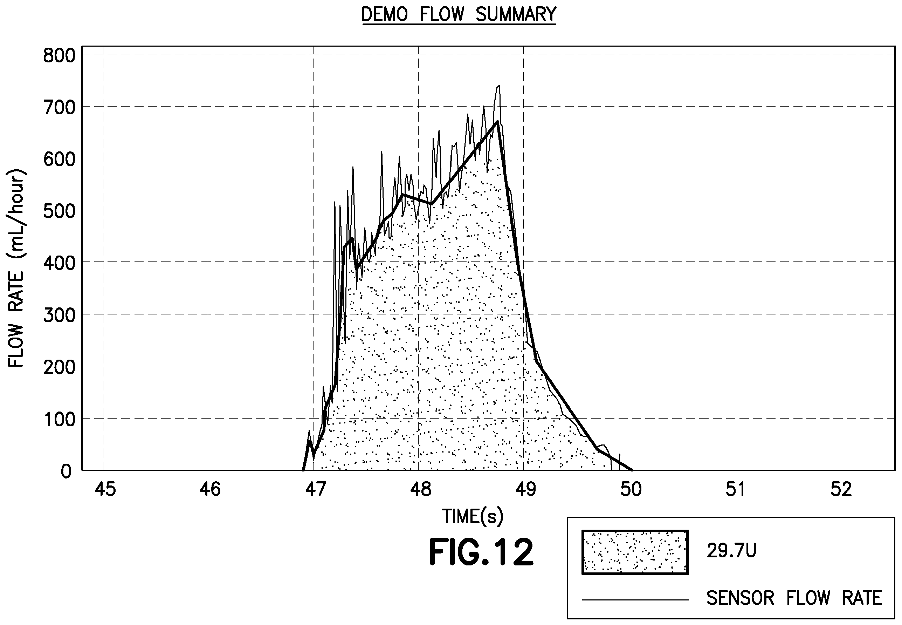

According to exemplary embodiments of the invention, TTOF flow sensing uses a central heating element with offset thermal sensing elements. The offset of each sensing element is preferably, but not necessarily, symmetrical on either side of the heating element. A time varying signal of known amplitude, frequency, shape and phase is applied to the central heater. The heat signal diffuses through the fluid toward the sensors, where it is detected with both reduced amplitude and a shifted phase relative to the drive signal. The amplitude signal corresponds to calorimetric sensing while the phase shift signal corresponds to time of flight sensing. Without flow, the thermal conduction zone around the heater is symmetric, as shown in FIG. 10. The electronics in the durable portion 106 senses balanced signals from the upstream and downstream sensors. The common signal seen by both sensors if filtered out by the electronics and the electronics calculates a no-flow condition for the sensor. With flow, the thermal zone is distorted by fluid convection, as shown in FIG. 11. The thermal signal is unbalanced, and the electronic signal seen on the downstream sensing element is shifted in phase (time) and amplitude relative to the input and relative to the upstream sensor. Advantageously, sensor signals from both upstream and downstream sensor traces are sampled throughout the flow range expected during an injection event. The shift in sensor signals is read by the durable portion 106 electronics and converted into an instantaneous flowrate of insulin by referencing a stored calibration curve or table. By sampling the instantaneous flowrate at precise and frequent time intervals, the total volume delivered can be calculated for each dose event. FIG. 12 illustrates an exemplary dose event, and the related data captured by the flow sensor to calculate the volume of the dose.

Several dose tracking insulin pens are currently available on the market. These pens track and monitor the motion of the pen mechanism to determine the dose delivered. Conventional pens use a small display to communicate intended dose volume of recent injections. Some newer models also incorporate wireless communication to a smart phone. Tracking the pen injection mechanism can fail to correctly monitor dose received by the user, as the pen mechanization has inherent error, which can be additive to the error in the sensing apparatus. Additionally, user errors, such as withdrawing the pen from the injection site before the dose is completely delivered into the tissue or not recognizing failures with system components, e.g. a pinched or clogged pen needle, can all contribute to not delivering the intended dose. Unlike conventional insulin pens, exemplary embodiments of the invention advantageously utilize TTOF sensing to measure the time and changing insulin flowrate profile actually delivered from the pen, allowing for more complete and accurate information on the actual dose delivered.

TTOF sensors measure the velocity of fluid passing the sensor. Accordingly, the volume of fluid passing through a flow sensor utilizing TTOF to measure fluid velocity is dependent upon the cross sectional area of the flow channel through which the fluid flows. As discussed above, injection molded parts are limited in how accurate the flow channel geometry can be made, and in how variable they are from part to part. Embodiments of the present invention improve upon the above described disposable portion that interfaces between the insulin pen and a pen needle to measure flow from the insulin pen, through the disposable portion, and into the pen needle to the patient. Metal tubes can be manufactured relatively inexpensively, and with highly accurate inner diameters, such as is known for needles and metal cannulas. Embodiments of the present invention advantageously utilize a metal cannula as the flow channel through which fluid flows for flow rate measurement and dose recording.

Exemplary embodiments of the invention form the sensor onto a glass substrate. The glass substrate has low thermal conductivity and structural rigidity to prevent deformation of the sensor during a dosing event. Forming the sensor surface on a glass substrate is preferred in the liquid medicament flow application, since preferably one sensor surface is exposed to the medicament within a flow channel, and overcomes the limitations described above in connection with thin membrane or bridge structures used on gaseous applications where pressures imparted onto the sensor are not a concern.

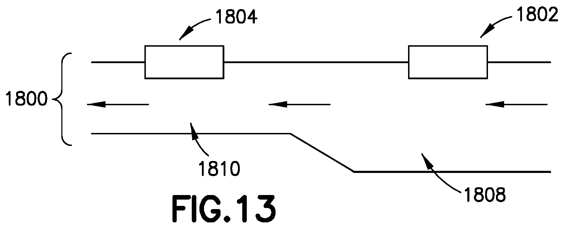

In another exemplary embodiment of the invention, multiple sensor chips are used in tandem to increase the dynamic range of the sensor. FIG. 13 illustrates an alternate flow channel 1800 having a first sensor 1802 and a second sensor 1804 downstream from the first sensor 1802. The flow channel 1806 is provided with two different cross section zones corresponding to the two sensor chips. The first sensor 1802 can be placed in a relatively larger flow cross section 1808 within the flow channel 1806. This sensor will have better resolution at higher flow rates due to relatively lower flow rates. The second sensor 1804 is placed downstream of the first sensor 1802 in an area of the flow channel 1806 having a smaller cross section 1810. The second sensor 1804 will have better resolution at lower flow rates due to relatively higher flow rates. Of course, one of ordinary skill in the art will readily appreciate that more than two sensors can be used to further improve dynamic range if necessary. The dose sensing algorithm determines the appropriate sensor pair to power and read signals from based on measured flow conditions and trends.

## Claims

1. A flow sensor assembly (104) comprising:
a flow manifold (1204) comprising:
a proximal end (120) shaped for connection to an insulin pen (102), the proximal end having a piercing member (122) extending therefrom;
a threaded distal end (114) shaped for connection to a pen needle (110), the threaded distal end having a septum (112);
a metal flow channel extending from the proximal end to the distal end,
a flow sensor (1200) for sensing the velocity of fluid flowing in the metal flow channel
**characterized in that**
the flow manifold is injection molded onto the metal flow channel and the metal flow channel has a sensor window formed prior to injection molding, and the flow sensor is arranged in the sensor window.

2. The flow sensor assembly (104) of claim 1, wherein the metal flow channel has a circular cross section.

3. The flow sensor assembly (104) of claim 1, wherein the metal flow channel has a rectangular cross section.

4. The flow sensor assembly (104) of claim 1, wherein the metal flow channel has a rectangular cross section and a circular cross section, and a smooth transitions between the rectangular cross section and the circular cross section.

5. A method of manufacturing a flow sensor assembly (104) according to any of claims 1-4 comprising the steps of:
forming a metal flow channel having a predetermined internal cross sectional area;
forming a sensor window (1304) in a side wall of the metal flow channel;
injection molding a flow manifold (1204) around the metal flow channel after forming the sensor window; and
mounting a flow sensor (1200) within the sensor window.

6. The method of claim 5, wherein the step of injection molding a flow manifold (1204) comprises:
forming an insulin pen (102) connector at a proximal end of the flow manifold (1204).

7. The method of claim 5, wherein the step of injection molding a flow manifold (1204) comprises:
forming a threaded pen needle (110) connector at a distal end of the flow manifold (1204).

8. The method of claim 7, further comprising: installing a septum (112) in the distal end of the flow manifold (1204).

## Patentansprüche

1. Durchflusssensoranordnung (104) mit:
einen Durchflussverteiler (1204) mit:
einem proximalen Ende (120), das zur Verbindung mit einem Insulin-Pen (102) ausgebildet ist, wobei das proximale Ende ein sich davon erstreckendes Durchstechelement (122) aufweist;
einem mit einem Gewinde versehenen distalen Ende (114), das zur Verbindung mit einer Pen-Nadel (110) ausgebildet ist, wobei das mit einem Gewinde versehene distale Ende ein Septum (112) aufweist;
einem Durchflusskanal aus Metall, der sich vom proximalen Ende zum distalen Ende erstreckt,
einem Durchflusssensor (1200) zum Erfassen der Geschwindigkeit des in dem Durchflusskanal aus Metall fließenden Fluids,
**dadurch gekennzeichnet,**
**dass** der Durchflussverteiler auf den Durchflusskanal aus Metall gespritzt ist, und dass der Durchflusskanal aus Metall ein vor dem Spritzgießen geformtes Sensorfenster aufweist und der Durchflusssensor im Sensorfenster angeordnet ist.

2. Durchflusssensoranordnung (104) nach Anspruch 1, wobei der Durchflusskanal aus Metall einen kreisförmigen Querschnitt aufweist.

3. Durchflusssensoranordnung (104) nach Anspruch 1, wobei der Durchflusskanal aus Metall einen rechteckigen Querschnitt aufweist.

4. Durchflusssensoranordnung (104) nach Anspruch 1, wobei der Durchflusskanal aus Metall einen rechteckigen Querschnitt und einen kreisförmigen Querschnitt sowie einen stufenlosen Übergang zwischen dem rechteckigen Querschnitt und dem kreisförmigen Querschnitt aufweist.

5. Verfahren zur Herstellung einer Durchflusssensoranordnung (104) nach einem der Ansprüche 1-4, das die folgenden Schritte umfasst:
Bilden eines Durchflusskanals aus Metall mit einer vorgegebenen inneren Querschnittsfläche;
Bilden eines Sensorfensters (1304) in einer Seitenwand des Durchflusskanals aus Metall;
Spritzgießen eines Durchflussverteilers (1204) um den Durchflusskanal aus Metall nach dem Bilden des Sensorfensters; und
Montieren eines Durchflusssensors (1200) innerhalb des Sensorfensters.

6. Verfahren nach Anspruch 5, wobei der Schritt des Spritzgießens eines Durchflussverteilers (1204) umfasst:
Ausbilden eines Insulin-Pen (102)-Konnektors an einem proximalen Ende des Durchflussverteilers (1204).

7. Verfahren nach Anspruch 5, wobei der Schritt des Spritzgießens eines Durchflussverteilers (1204) umfasst:
Ausbilden eines Pen-Nadelgewindekonnektors (110) an einem distalen Ende des Durchflussverteilers (1204).

8. Verfahren nach Anspruch 7, ferner mit dem Schritt: Einsetzen eines Septums (112) in das distale Ende des Durchflussverteilers (1204).

## Revendications

1. Ensemble capteur d'écoulement (104) comprenant :
un collecteur d'écoulement (1204) comprenant :
une extrémité proximale (120) formée pour être connectée à un stylo à insuline (102), l'extrémité proximale présentant un élément de perçage (122) s'étendant à partir de celle-ci ;
une extrémité distale filetée (114) formée pour être connectée à une aiguille de stylo (110), l'extrémité distale filetée présentant un septum (112) ;
un canal d'écoulement métallique s'étendant de l'extrémité proximale à l'extrémité distale ;
un capteur d'écoulement (1200) permettant de détecter la vitesse du fluide s'écoulant dans le canal d'écoulement métallique ;
**caractérisé en ce que**
le collecteur d'écoulement est moulé par injection sur le canal d'écoulement métallique et le canal d'écoulement métallique présente une fenêtre de capteur formée avant le moulage par injection, et le capteur d'écoulement est agencé dans la fenêtre de capteur.

2. Ensemble capteur d'écoulement (104) de la revendication 1, dans lequel le canal d'écoulement métallique présente une section transversale circulaire.

3. Ensemble capteur d'écoulement (104) de la revendication 1, dans lequel le canal d'écoulement métallique présente une section transversale rectangulaire.

4. Ensemble capteur d'écoulement (104) de la revendication 1, dans lequel le canal d'écoulement métallique présente une section transversale rectangulaire et une section transversale circulaire, et des transitions lisses entre la section transversale rectangulaire et la section transversale circulaire.

5. Procédé de fabrication d'un ensemble de capteur d'écoulement (104) selon l'une quelconque des revendications 1 à 4, comprenant les étapes consistant à :
former un canal d'écoulement métallique présentant une aire de section transversale interne prédéterminée ;
former une fenêtre de capteur (1304) dans une paroi latérale du canal d'écoulement métallique ;
mouler par injection un collecteur d'écoulement (1204) autour du canal d'écoulement métallique après formation de la fenêtre de capteur ; et
monter un capteur d'écoulement (1200) dans la fenêtre de capteur.

6. Procédé de la revendication 5, dans lequel l'étape de moulage par injection d'un collecteur d'écoulement (1204) comprend l'étape consistant à : former un connecteur de stylo à insuline (102) au niveau d'une extrémité proximale du collecteur d'écoulement (1204).

7. Procédé de la revendication 5, dans lequel l'étape de moulage par injection d'un collecteur d'écoulement (1204) comprend l'étape consistant à : former un connecteur fileté d'aiguille de stylo (110) au niveau d'une extrémité distale du collecteur d'écoulement (1204).

8. Procédé de la revendication 7, comprenant en outre l'étape consistant à : installer un septum (112) dans l'extrémité distale du collecteur d'écoulement (1204).
